**Europäisches Patentamt**

**European Patent Office**

**Office européen des brevets**

(19)

(11) Veröffentlichungsnummer : **0 170 715 B1**

(12) **EUROPÄISCHE PATENTSCHRIFT**

(45) Veröffentlichungstag der Patentschrift :
**13.04.88**

(51) Int. Cl.⁴ : **A 61 M 11/06**, A 61 M 15/00

(21) Anmeldenummer : **84109456.8**

(22) Anmeldetag : **09.08.84**

(54) **Zerstäubervorrichtung.**

(43) Veröffentlichungstag der Anmeldung :
**12.02.86 Patentblatt 86/07**

(45) Bekanntmachung des Hinweises auf die Patenterteilung : **13.04.88 Patentblatt 88/15**

(84) Benannte Vertragsstaaten :
**AT BE CH DE FR GB IT LI LU NL SE**

(56) Entgegenhaltungen :
**FR-A- 1 156 698**
**GB-A- 269 100**
**US-A- 3 010 910**

(73) Patentinhaber : **Brugger, Inge**
**Prinz-Karl-Strasse 50a**
**D-8130 Starnberg (DE)**

**Brugger, Stephan**
**Erztalstrasse 21**
**D-8137 Berg (DE)**

(72) Erfinder : **Der Erfinder hat auf seine Nennung verzichtet**

(74) Vertreter : **Hoffmann, Klaus, Dr. rer. nat. et al**
**Hoffmann . Eitle & Partner Patentanwälte Arabellastrasse 4**
**D-8000 München 81 (DE)**

Jouve, 18, rue St-Denis, 75001 Paris, France

## Beschreibung

Die Erfindung betrifft eine Zerstäubervorrichtung nach dem Oberbegriff von Patentanspruch 1.

Eine derartige Zerstäubervorrichtung ist in der DE-C 1 147 355 beschrieben. Mit der vorbekannten Zerstäuberdüse erreicht man eine zufriedenstellende Zerstäubung auch von Flüssigkeiten bis zu einer Viskosität von etwa 70 cp bei einem Gasdruck von nur etwa 0,6 bar.

In dem Bestreben, immer leistungsfähigere Inhaliergeräte zu schaffen, die eine verbesserte Zerstäuberwirkung bei gleichem Gasdruck zeigen, hat man Versuche auch mit gegenüber der vorbekannten Düse abgewandelten Düsen vorgenommen und durch moderne klinische Methoden den intrathorakalen (lungengängigen) Aerosolanteil gemessen. Dabei handelt es sich um denjenigen Anteil, dessen Tröpfchengröße in einem Spektrum von etwa 0,5 bis 5 μ Durchmesser liegt und der bis in die feinsten Verästelungen der Lunge beim Einatmen gelangt.

Ein derartiges Inhaliergerät ist in dem DE-U 8 302 105 beschrieben. Bei diesem Gerät ist die Verneblerdüse mit einem axialgerichteten mittigen Auslaß ausgebildet und diesem Auslaß ein Pralldorn mit einer Prallstirnfläche gegenübergestellt, an der das Aerosol erzeugt wird. Zusätzlich kann bei dieser Ausführung um den Pralldorn herum auch ein koaxialer Prallhelm angeordnet sein, in dem in Höle der Verneblerdüse fensterartige Auslaßöffnungen für das an der Prallfläche erzeugte Aerosol vorgesehen sind. Diese Fenster erstrecken sich über etwa die Hälfte oder mehr des Prallhelmumfangs. Diese Vorrichtung weist keinen zentralen Zuluftkamin auf. Die in der Einatemphase notwendige Zuluft wird bei einem nach diesem DE-U gefertigten Inhaliergerät durch Schlitze am Rand in dem Verbindungsflansch zwischen dem Behälter und der Verneblerhaube zugeführt. An dem Prallhelm werden größere und mittlere Flüssigkeitströpfchen aufgefangen und aus dem Nebel ausgesondert. Eine Erhöhung des Anteils lungengängiger Aerosolteilchen erfolgt dabei nicht, da der Aerosolstrom von der zentralen waagerechten Prallfläche im wesentlichen waagerecht nach außen geleitet wird und hierbei allenfalls durch zusätzliche Flüssigkeitströpfchen abgelenkt wird, die aus dem sich konisch erweiternden Ausgang der Verneblerdüse austreten und die Prallfläche nicht mehr treffen.

Es ist die Aufgabe der Erfindung, eine Zerstäubervorrichtung der vorgenannten Art so zu verbessern, daß bei vorgegebenem Gasdruck eine möglichst große Aerosolmenge und daraus wieder ein möglichst hoher Anteil von intrathorakalen (lungengängigen) Aerosolpartikeln, d. h. von Teilchen in einer Größe zwischen etwa 0,5 und 5,5 μ Durchmesser erzeugt wird.

Dieses Ziel wird gemäß der Erfindung mit einer Zerstäubervorrichtung erreicht, die sich durch die Merkmale des Kennzeichens von Patentanspruch 1 auszeichnet. Durch die Anbringung eines zylindrischen Einsatzes mit einem sich schräg nach außen und abwärts in den Verneblerraum erstreckenden Prallschirm am unteren Ende des koaxialen Zuluftkamins werden die mit hoher Energie auf die innere Mantelfläche des Prallschirms auftreffenden Aerosolteilchen nicht nur umgelenkt oder in Tropfenform abgeschieden, sondern weiter zerkleinert, so daß der Anteil der lungengängigen Aerosolfraktion ganz erheblich gesteigert wird.

Vorzugsweise erstreckt sich der Prallschirm kegelig nach außen, wobei er einen Winkel von etwa 120° gegenüber der Mantelfläche einnehmen kann. Er kann jedoch auch glockenförmig, kugelig oder parabolisch geformt sein, wobei im letzteren Fall die untere Mantelfläche des Prallschirmes noch unter einem kleinen Winkel gegenüber der Mantelfläche des Zuluftkamins nach außen verläuft. Dabei erstreckt sich der Außenrand des Schirms gegen die Innenwandung der Verneblerhaube und reduziert so den Ringraum um den Zuluftkamin ; bei einem Ausführungsbeispiel auf etwa ein Drittel des freien Durchlaßquerschnittes. Die an den schrägen Prallflächen des Gasstromsteuers erzeugten und schräg nach oben abgelenkten Aerosolpartikel gelangen so in den Bereich der inneren Mantelfläche des Schirmes, wo größere Partikel durch den Aufprall nochmals zerkleinert werden. Dadurch wird der Gesamtanteil der lungengängigen Aerosolfraktion im gewünschten Spektrum zwischen 0,5 bis 5,5 μ vergrößert. Durch die Unterkante des Prallschirms erfolgt zusätzlich auch eine Ausfilterung und Abscheidung von zu großen Teilchen. Gelangen zu große Teilchen in den Ringraum um den Zuluftkamin, so werden sie dort bei der üblicherweise senkrechten Handhabung des Zerstäubers infolge ihrer Schwerkraft augeschieden, zum Zerstäubungsgut zurückgeleitet und gelangen nicht aus der Verneblerhaube in den Nebelaustrittsstutzen.

Bei einer Weiterbildung der Zerstäubervorrichtung kann die Gesamtbauhöhe der Einrichtung zur Erzielung eines kompakten Verneblers dadurch reduziert werden, daß der Aerosolstrom in der Verneblerhaube um den Zuluftkamin herum über eine Art Labyrinth mit mindestens einer zusätzlichen Abrißkante herumgeleitet wird, wodurch zu große Tröpfchen nicht nur infolge der Schwerkraft, sondern auch durch ihren Kontakt an einer derartigen Schikane infolge der Umleitung abgeschieden und dem Sammelbehälter wieder zugeführt werden.

Bei axialsymmetrischer Bauweise des Zerstäubers kann in einfacher Weise eine zusätzlicher Einsatz zur Umlenkung des Aerosolstromes zwischen Behälter und Haube vorgesehen sein, wodurch man die angestrebte Verringerung der Bauhöle des koaxialen Zuluftkamins erreicht. Der zusätzliche Einsatz kann von einem Bauteil mit einem zylindrischen Randflansch und einem sich nach oben in den Verneblerraum etwa kegel-

stumpfförmig verjüngenden Wandteil gebildet sein, dessen Innenrand als Abrißkante ausgebildet ist. Dieser Einsatz kann durch entsprechende Gestaltung der miteinander in Eingriff stehenden Ränder des Behälters und der Haube zwischen diesen gehalten sein.

Die beiden bei dieser Ausführungsform geschaffenen Abrißkanten für die Ausfilterung von zu großen Aerosoltröpfchen und zwar die Außenkante des sich nach unten erstreckenden Prallschirms und die Innenkante des genannten Einsatzes erlauben eine erhebliche Reduzierung der Bauhöle der erfindungsgemäßen Zerstäubervorrichtung und ermöglichen so eine nahezu kugelige Gestaltung, die beispielsweise den Einsatz eines so geschaffenen Zerstäuberkopfes in einen angepassten Handgriff ermöglicht.

Weitere Einzelheiten der Erfindung ergeben sich aus der Beschreibung zweier in der Zeichnung dargestellter Ausführungsbeispiele.

Figur 1 ist ein Mittellängsschnitt durch eine erste Ausführungsform der Zerstäubervorrichtung ;

Figur 2 zeigt einen vergrößerten Ausschnitt des mittleren Teils der Vorrichtung von Fig. 1 ;

Figur 3 ist ein Mittellängsschnitt durch eine zweite Ausführungsform der Zerstäubervorrichtung.

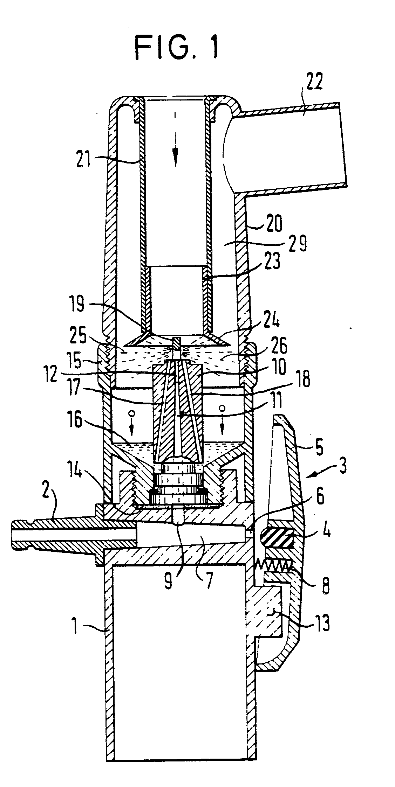

Die in Fig. 1 gezeigte Zerstäubungsvorrichtung besteht im wesentlichen aus dem zylindrischen Vernebleruntereil 1 mit Anschlußstutzen 2 für die Druckgasleitung und dem gegenüber dem Anschlußstutzen 2 angeordneten Tasthebel 3 mit einem Dichtungseinsatz 4 zum Verschließen der Auslaßöffnung 6 im Zuleitungskanal 7.

Der Tasthebel 3 kann vom Benutzer in einfacher Weise aus seiner in Fig. 1 gezeigten Außerbetriebsstellung unter Überwindung der Kraft der Feder 8 mit dem Vorderteil des Tasthebels 3 in Anlage zur Oberfläche des Verneblers gebracht werden, wobei der Dichteinsatz 4 sich auf die Auslaßöffnung 6 legt und so den Zuleitungskanal für das Druckgas absperrt und dieses durch die Querbohrung 9 in die zentrale Druckgasleitung 11 des Düsenkopfes 10 umleitet. Der Tasthebel 3 dient zur Unterbrechung des Druckgasstromes in den Ausatemphasen des Benutzers, in denen kein Aerosol erzeugt werden soll. Er kann jedoch auch für den Dauerbetrieb des Inhalationsgerätes in Anlage am Gehäuse fixiert werden, indem der Tasthebel mittels eines ihn in seinem hinteren Bereich übergreifenden kastenförmigen Schiebers dadurch in seine Betriebsstellung verschwenkt wird, daß der Schieber mittels zweier in seinen Seitenwandungen vorgesehener in den Zapfen 13 geführter Langlöcher über den Tasthebel 3 geschoben wird.

In das Vernebleruntereil 1 ist in bekannter Weise unter Zwischenschaltung eines elastischen Dichtungsringes 14 der gleichfalls zylindrische Behälter 15 zur Aufnahme des zu zerstäubenden Gutes 16 eingeschraubt. Im Behälter 15 ist auch die Zerstäuberdüse, bestehend aus dem Düsenkopf 10 mit der zentralen Druckgasleitung 11 und den beiden seitlichen Ansaugkanälen 17 und 18 für das zu zerstäubende Gut 16 und dem Gasstromsteuer 19 untergebracht. Die zentrale Druckgasleitung 11 verjüngt sich gegen das obere Ende des Düsenkopfes zu und endet in der schmalen Düsenbohrung 12, welche unterhalb des Gasstromsteuers 19 aus dem Düsenkopf ausmündet.

Auf dem Behälter 15 ist die zylindrische Verneblerhaube 20 aufgeschraubt. Die Verneblerhaube 20 enthält einen koaxialen Zuluftkamin 21, der sich bis dicht über das Gasstromsteuer 19 in den Innenraum des Verneblers hineinerstreckt. Im oberen Teil ist an die Verneblerhaube 20 der Nebelaustrittstutzen 22 angesetzt, an den ein in der Zeichnung nicht dargestelltes Mundstück ansetzbar ist.

Der Zuluftkamin 21 trägt an seinem unteren Ende einen zylindrischen Einsatz 23 mit einem Prallschirm 24. An diesem wird zumindest ein Teil der größeren Partikel des an den Schrägflächen des Gasstromsteuers 19 erzeugten Aerosols zersplittert und so weiter zerkleinert.

Bei der vorbekannten Konstruktion gemäß DE-PS 1 147 355 war man davon ausgegangen, daß bei entsprechender Dimensionierung des Durchmessers der Verneblerhaube bei vorgegebenem Überdruck der Druckgasquelle die Innenwandung der Verneblerhaube als Prall- und auch Sammelfläche für zu große Tropfen des Aerosolstromes ausreicht. Entsprechende Messungen der lungengängigen Aerosolstrommenge mit radioaktive markiertem zu zerstäubenden Gut haben ergeben, daß in erwünschter Weise eine relativ große Menge Aerosol vom Benutzer in der Zeiteinheit eingeatmet wird und daß ein erheblicher Anteil davon auch intrathorakal resorbiert wird (vgl. Zeitschrift der Stiftung Warentest 1983, Nr. 6, S. 32-37).

Dieser intrathorakale Anteil beträgt in dem genannten Vergleichstest für das Pari-Therapiegerät beispielsweise ca. 40 % , während er bei Einsatz der erfindungsgemäßen Zerstäubervorrichtung erstaunlicherweise nahezu verdoppelt ist.

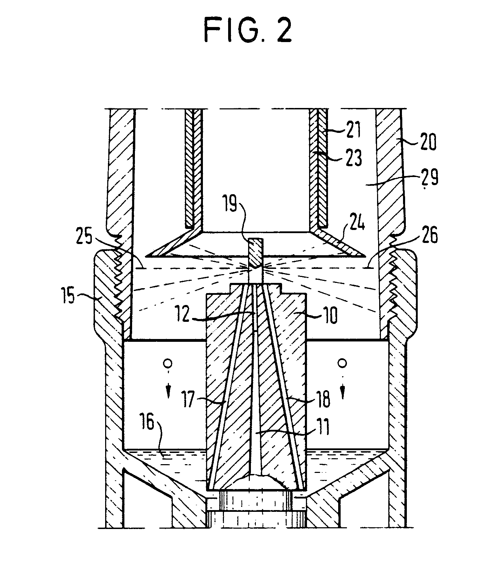

Anhand von Fig. 2, die einen vergrößerten Querschnitt des eigentlichen Zerstäuberteils mit dem Prallschrim 24 darstellt, wird die Wirkungsweise des neuen Zerstäubers im folgenden erläutert.

An den in Fig. 2 nicht dargestellten Anschlußstutzen 2 wird eine Druckgasquelle, z. B. ein Kompressor, der den erforderlichen Überdruck erzeugt (ca. 0,6 bar) angeschlossen. Das Druckgas steigt durch die zentrale Druckgasleitung 11 und die Düsenbohrung 12 bis zur Austrittsöffnung im Düsenkopf 10. Durch die Verjüngung der zentralen Druckgasleitung im oberen Bereich des Düsenkopfes 10 erhält die Druckluft eine hohe Austrittsgeschwindigkeit. Beim Austritt der Luft aus der Düsenbohrung 12 wird durch die benachbarten Ansaugkanäle 17, 18 das Zerstäubungsgut 16 aus dem unteren Teil des Behälters 15 nach oben gesaugt und an den schräggestellten Prallflächen des Gasstromsteuers 19 zerkleinert und verteilt. Der übliche Keilwinkel des Gasstromsteuers 19 beträgt etwa 120°, so daß sich beiderseitig zwischen der oberen Ebene des Düsenkopfes 10 und der Verlängerung der Keilflächen des Gasstromsteuers 19 je ein Aerosolfächer 25, 26 über einen Winkelbereich von etwa 30° nach oben ausbildet. Die Ausbreitung dieser Aerosolfächer nach unten ist durch die Oberfläche des Düsenkopfes 10 begrenzt. Der zylindrische Einsatz 23 mit dem Prallschirm 24 bewirkt nun, daß ein Teil des Sprühgutes im Bereich der Aerosolfächer 25, 26 mit erheblicher kinetischer Energie auf die Innenwandung des Prallschirmes 24 auftrifft und dabei wenigstens größere Tröpfchen nochmals zerkleinert werden. So erhält man eine zusätzliche Aerosolmenge in dem gewünschten Bereich zwischen 0,5 und 5,5 µ Partikelgröße, die intrathorakal aufgenommen werden kann.

Durch die Anordnung des zusätzlichen Prallschirmes 24 wird die wirksame Fläche des Innenmantels der Verneblerhaube 20 als Prallfläche für die Aerosolfächer 25, 26 zwar begrenzt, die intensivere Wechselwirkung zwischen der Innenfläche des Prallschirms 24 und den Fächern kompensiert diesen Verlust aber nicht nur, sondern scheint hier gerade den gewünschten Effekt auszumachen.

Der Prallschirm 24 bewirkt eine Begrenzung des Ringspaltes zwischen der Außenwandung des Zuluftkamins 21 und der Innenwandung der Verneblerhaube 20 ; dadurch werden bereits in diesem Bereich zu große Tröpfchen an der Kante des Prallschirmes 24 und an der Innenwandung der Verneblerhaube abgeschieden und laufen in den Behälter 15 zum Zerstäubungsgut 16 zurück.

Es versteht sich, daß anstelle des zylindrischen Einsatzes 23 mit Prallschirm 24 auch der koaxiale Kamin 21 an seinem Ende mit einem entsprechenden Prallschirm 24 versehen sein kann. Die Zweiteiligkeit hat jedoch den Vorteil, daß die beiden Teile beim Reinigen des Gerätes in einfacher Weise auseinandergeschoben werden können und sich so der Kamin 21 in einfacher Weise aus der Verneblerhaube 20 nach oben entfernen läßt.

Bei der Ausführungsform des Verneblers nach den Figuren 1 und 2 erstreckt sich der Prallschirm 24 unter einem Winkel von etwa 120° von der Mantelfäche des Kamins 21 nach außen und zwar soweit, daß sein Außenrand bei etwa zwei Drittel des freien Kreisringdurchmessers von innen nach außen zwischen dem koaxialen Kamin 21 und der Verneblerhaube 20 zu liegen kommt.

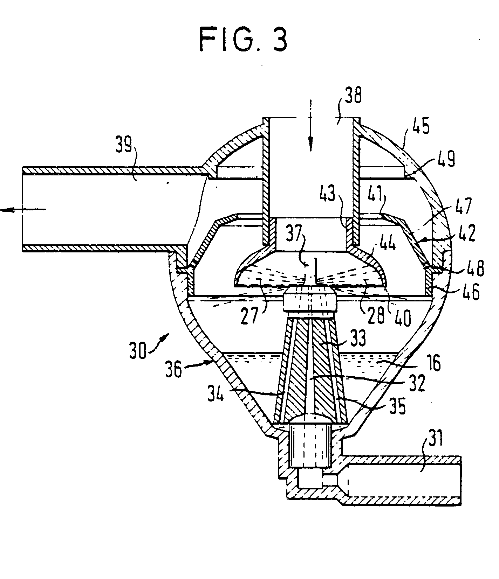

Eine zweite Ausführungsform der Erfindung ist in Fig. 3 dargestellt. Dabei handelt es sich um die vergrößerte Ansicht eines Zerstäuberkopfes, bei welchem der erfindungsgemäße Prallschirm 44 in ähnlicher Weise wie bei dem Ausführungsbeispiel nach den Figuren 1 und 2 realisiert ist ; der Schirm ist im Querschnitt hier jedoch nicht kegelig, sondern parabolisch ausgebildet. Der gezeigte Zerstäuberkopf ist Teil eines an eine Druckgasquelle anschliessbaren Handgerätes für Inhalationszwecke, wobei der zugehörige den Zerstäuberkopf 30 umschliessende Handgriff nicht dargestellt ist. Der Zerstäuberkopf 30 wird über den Anschlußstutzen 31 an eine Druckgasquelle angeschlossen. Die Druckgaszufuhr kann durch ein in der Zeichnung nicht dargestelltes separat aufbringbares Unterbrecherventil geregelt werden. Beim Austritt des Druckgases aus der zentralen Druckgasleitung 32 im Düsenkopf 33 wird von den benachbarten Ansaugkanälen 34, 35 das Zerstäubungsgut 16 aus dem Behälter 36 nach oben gesaugt und am Luftstromsteuer 37 zerkleinert. Die Druckgasleitung weist in ihrem oberen Teil gleichfalls eine verjüngte schmale Düsenbohrung zur Beschleunigung des Druckgases auf. Aufgrund der hohen Austrittsgeschwindigkeit entstent in diesem Bereich ein Unterdruck, der dafür sorgt, daß in gleicher Weise wie beim ersten Ausführungsbeispiel über den koaxialen Kamin 38 Zuluft angesaugt wird, so daß sich im Aerosolaustrittsstutzen 39 die gewünschte Luftmenge einstellt.

Die Wirkungsweise des Zerstäubers gemäß Fig. 3 ist ähnlich wie die gemäß den Figuren 1 und 2 : nach der Ausbildung des Aerosols am Gasstromsteuer 37 gelangt eine Teil der Aerosolpartikel der Fächer 27, 28 an die Innenseite des Prallschirmes 44, der am Ende des zylindrischen Einsatzes 43 ausgebildet ist und wird dadurch noch weiter zerkleinert. Größere Teilchen tropfen von der Kante 40 des Prallschirms 44 ab und gelangen so wieder zum Zerstäubungsgut 16 im Behälter 36 zurück.

Die Wirkung des relativ hohen Ringkamins in der Verneblerhaube 20 um den koaxialen Kamin 21 bei der ersten Ausführungsform wird bei der Variante gemäß Fig. 3 kompensiert durch eine Art Labyrinthführung des Aerosolstromes bis zum Austrittsstutzen 39, wobei dieser neben der bereits genannten Kante 40 noch eine weitere Kante 41 eines im Behälter 36 angeordneten Einsatzes 42 umströmen muß. Dabei werden weitere größere Partikel aus dem Aerosolstrom ausgefiltert, die an der Innenwandung des Einsatzes 42 entlang wieder in den Behälter 36 zurücklaufen. Das so von zu großen Partikeln gefilterte Aerosol verläßt den Zerstäuberkopf durch den Austrittsstutzen 39 und enthält gleichfalls einen hohen Anteil von lungengängigem Aerosol.

Die kugelige Konfiguration gemäß Fig. 3 hat gegenüber dem in Fig. 1 gezeigten den Vorteil einer geringen Bauhöhe. Der Zerstäuberkopf läßt sich daher in einfacher Weise, wie bereits eingangs erwähnt, in einem Handgriff unterbringen.

Der Einsatz 42 ist von einem axialsymmetrischen Bauteil mit einem zylindrischen Randflansch 46 und einem sich nach oben in den Verneblerraum kegelstumpfförmig verjüngenden Wandteil 47 gebildet, dessen Rand unter Ausbildung der zweiten Kante 41 soweit nach innen gezogen ist, daß im zusammengebauten Zustand nur noch eine relativ schmaler Ringraum zwischen der Außenwandung des Kamins 38 und der Kante 41 verbleibt, so daß größere Tröpfchen im Bereich der Kante 41 abgeschieden werden und in der beschriebenen Weise wieder in den Behälter 36 zurücklaufen. Dabei besitzt der Einsatz 42 zweckmäßigerweise eine nach außen vorspringende Lippe 48 zwischen dem Randflansch 46 und dem Wandteil 47. Diese Lippe dient zur Fixierung des Einsatzes 42 an einem entsprechend ausgebildeten Rand des Behälters 36, so daß beim Zusammenbau der Zerstäubervorrichtung, d. h. beim Aufsetzen der Haube 45 auf den Behälter 36 der Einsatz 42 automatisch zwischen diese beiden Teile geklemmt und in dieser Lage fixiert ist.

Die Austrittsstutzen 22 bzw. 39 für das Aerosol sind so gestaltet, daß verschiedene Aufsätze für spezielle Inhalationen aufsteckbar sind.

Als Material für sämtliche Teile des Verneblers kommt ein geeigneter spritzfähiger Kunststoff bekannter Art zum Einsatz.

**Patentansprüche**

1. Vorrichtung zum Zerstäuben, Verteilen und Vermischen von flüssigen und pulverförmigen Stoffen mittels eines Druckgasstromes, insbesondere für die Erzeugung von Aerosolen für Inhalationszwecke, bestehend aus einem Behälter (15) für das Zerstäubungsgut (16) und einer auf den Behälter aufsetzbaren Verneblerhaube (20) und mit einem koaxialen Kamin (21) für den Eintritt von Zuluft in den Zerstäubungsraum, bei welcher das aus einem zentral im Behälter angeordneten Düsenkopf (10) austretende gasförmige Druckmittel das Zerstäubungsgut aus der Düse benachbarten Ansaugkanälen (17, 18) ansaugt und bei welcher gegenüber der Düsenmündung im Austrittskegel des Druckgases eine Gasstromsteuer (19) vorgesehen ist, welches auf seiner der Düsenöffnung gegenüberliegenden Seite keilförmig ausgebildet ist und welches nahe der Mündungsebene der Düsenöffnung für das Druckmittel liegt, dadurch gekennzeichnet, daß ein zylindrischer Einsatz (23) mit einem sich nach außen und abwärts in den Verneblerraum erstreckenden Prallschirm (24) am unteren Ende des Zuluftkamins (21) angeordnet ist.

2. Vorrichtung nach Anspruch 1, dadurch gekennzeichnet, daß sich der Prallschirm kegelig nach außen erstreckt.

3. Vorrichtung nach Anspruch 1, dadurch gekennzeichnet, daß sich der Prallschirm (24) unter einem Winkel von 120° gegenüber der Mantelfläche des Zuluftkamins (21) nach außen erstreckt.

4. Vorrichtung nach Anspruch 1, dadurch gekennzeichnet, daß der Prallschirm glockenförmig bzw. kugelig rotationssymmetrisch ausgebildet ist.

5. Vorrichtung nach Anspruch 4, dadurch gekennzeichnet, daß die Mantelfläche des Prallschirms parabolisch geformt ist.

6. Vorrichtung nach Anspruch 5, dadurch gekennzeichnet, daß die untere Abrißkante des Prallschirms (24) in einem kleinen Winkel gegenüber der Mantelfläche des Zuluftkamins nach außen verläuft.

7. Vorrichtung nach den Ansprüchen 1 bis 6, dadurch gekennzeichnet, daß sich der Prallschirm (24) über etwa Drittel des freien Ringquerschnittes vom Rand des Zuluftkamins (21) nach außen in Richtung auf die Wandung der Verneblerhaube (20) erstreckt.

8. Vorrichtung nach einem der Ansprüche 1 bis 7, dadurch gekennzeichnet, daß zur Verringerung der Bauhöhe des koaxialen Zuluftkamins ein zusätzlicher Einsatz (42) zur Umlenkung des Aerosolstromes eingebaut ist.

9. Vorrichtung nach Anspruch 8, dadurch gekennzeichnet, daß der zusätzliche Einsatz (42) von einem axialsymmetrischen Bauteil mit einem zylindrischen Randflansch (46) und einem sich nach oben in den Verneblerraum etwa kegelstumpfförmig verjüngenden Wandteil (47) gebildet ist, dessen Rand unter Ausbildung einer Kante (41) nach innen gezogen ist und daß zwischen Randflansch (46) und Wandteil (47) eine nach außen vorspringende Lippe (48) zur Fixierung des Einsatzes an einem entspreschend ausgebildeten Rand des Behälters (36) vorgesehen ist.

10. Vorrichtung nach Anspruch 9, dadurch gekennzeichnet, daß am freien Rand des Prallschirmes (44) eine Kante (40) ausgebildet ist, daß am Innenrand des Einsatzes (42) eine weitere Kante (41) ausgebildet ist und daß an der Innenwandung der Haube (45) eine Tropfkante (49) angeformt ist, die zusammen mit den Kanten (40, 41) wirkt, indem an den so gebildeten Umlenkstellen für den Aerosolstrom größere Tröpfchen aus diesem abgesondert und in das Zerstäubungsgut (16) im Behälter (36) rückgeführt werden.

**Claims**

1. Device for atomising, dividing and mixing liquid and powdered materials by means of a compressed gas stream, in particular for generating aerosols for inhalation purposes, consisting of a receptacle (15) for the substance (16) to be atomised and a nebuliser cap (20) which can be mounted on the receptacle, and with a coaxial flue (21) for entry of input air into the atomising chamber, in which the gaseous pressure medium exiting from a nozzle head (10) arranged centrally in the receptable draws the atomising substance out of intake channels (17, 18) adjacent to the nozzle, and in which opposite the nozzle orifice in the output cone of the compressed gas is provided a gas stream controller (19) which is wedge-shaped on its side opposite the nozzle opening and which is located near the orifice plane of the nozzle opening for the pressure medium, characterised in that a cylindrical insert (23) with a baffle screen (24) extending outwardly and downwardly into the atomiser chamber is disposed at the lower end of the air inlet flue (21).

2. Device according to claim 1, characterised in that the baffle screen extends conically outwards.

3. Device according to claim 1, characterised in that the baffle screen (24) extends outwardly at an angle of 120° to the generated surface of the air inlet flue (21).

4. Device according to claim 1, characterised in that the baffle screen is rotationally symmetrical in a bell or spherical shape.

5. Device according to claim 4, characterised in that the generated surface of the baffle screen is parabolically shaped.

6. Device according to claim 5, characterised in that the lower sharp edge of the baffle screen (24) extends outwards at a low angle to the generated surface of the air inlet flue.

7. Device according to claims 1 to 6, characterised in that the baffle screen (24) extends outwards in a direction towards the wall of the atomiser cap (20) from the edge of the air inlet flue (21) over about two-thirds of the free ring cross-section.

8. Device according to any of claims 1 to 7, characterised in that to reduce the overall height of the coaxial air inlet flue, an additional insert (42) is fitted for deflecting the aerosol stream.

9. Device according to claim 8, characterised in that the additional insert (42) is composed of an axially symmetrical component with a cylindrical edge flange (46) and a wall portion (47) which tapers approximately frustoconically upwards into the atomiser chamber and whose edge is pulled inwards, forming an edge (41), and between edge flange (46) and wall portion (47) is provited an outwardly projecting lip (48) for fixing the insert on a correspondingly shaped edge of the receptacle (36).

10. Device according to claim 9, characterised in that at the free edge of the baffle screen (44) is formed an edge (40), at the inner edge of the insert (42) is formed an additional edge (41), and at the inner wall of the cap (45) is formed a drip edge (49) which cooperates with the edges (40, 41) in that, at the deflection points formed in this way for the aerosol stream, larger droplets are separated there from and returned to the atomising substance (16) in the receptacle (36).

## Revendications

1. Dispositif pour la pulvérisation, la distribution et le mélange de substances liquides et pulvérulentes au moyen d'un jet de gaz comprimé, en particulier pour la production d'aérosols à des fins d'inhalation, constitué par un réservoir (15) pour le produit de pulvérisation (16) et par un diffuseur (20) pouvant être installé sur le réservoir et comprenant une cheminée coaxiale (21) pour l'entrée de l'air dans la chambre de pulvérisation, dans lequel le fluide gazeux sous pression, sortant d'une tête de projection (10) placée au centre du réservoir, aspire le produit de pulvérisation des canaux d'aspiration (17, 18) voisins de la buse, et dans lequel, en face de l'embouchure de la buse, dans le cône de sortie du gaz comprimé, est prévue une commande de jet de gaz (19), qui est réalisée cunéiforme sur son côté situé en regard de l'ouverture de la buse et qui est placée à proximité du plan de l'embouchure de l'ouverture de la buse pour le fluide sous pression, caractérisé par le fait qu'une garniture cylindrique (23), comportant un écran déflecteur (24) s'étendant vers l'extérieur et vers le bas dans la chambre de pulvérisation, est disposée à l'extrémité inférieure de la cheminée de prise d'air (21).

2. Dispositif selon la revendication 1, caractérisé par le fait que l'écran déflecteur s'étend en forme de cône vers l'extérieur.

3. Dispositif selon la revendication 1, caractérisé par le fait que l'écran déflecteur (24) s'étend vers l'extérieur sous un angle de 120° par rapport à la surface latérale de la cheminée de prise d'air (21).

4. Dispositif selon la revendication 1, caractérisé par le fait que l'écran déflecteur est réalisé à la manière d'un corps de révolution en forme de cloche ou sphérique.

5. Dispositif selon la revendication 4, caractérisé par le fait que la surface latérale de l'écran déflecteur est de forme parabolique.

6. Dispositif selon la revendication 5, caractérisé par le fait que l'arête de décollement inférieure de l'écran déflecteur (24) s'étend vers l'extérieur sous un petit angle par rapport à la surface latérale de la cheminée de prise d'air.

7. Dispositif selon les revendications 1 à 6, caractérisé par le fait que l'écran déflecteur (24) s'étend vers l'extérieur sur environ les 2/3 de la section transversale annulaire libre, en partant du bord de la cheminée de prise d'air (21) et allant en direction de la paroi du diffuseur (20) du pulvérisateur.

8. Dispositif selon l'une des revendications 1 à 7, caractérisé par le fait que pour diminuer la hauteur hors tout de la cheminée de prise d'air coaxiale, il est prévu une garniture supplémentaire (42) pour dévier le jet d'aérosol.

9. Dispositif selon la revendication 8, caractérisé par le fait que la garniture supplémentaire (42) est formée par un élément de construction axisymétrique comprenant une bride marginale cylindrique (46) et une partie de paroi (47) pénétrant dans la chambre de pulvérisation en se rétrécissant sensiblement en forme de tronc de cône vers le haut et dont le bord s'étend vers l'intérieur en formant une arête (41), et qu'entre la bride marginale (46) et la partie de paroi (47), est prévue une lèvre (48) faisant saillie vers l'extérieur pour fixer la garniture sur un bord, réalisé en conséquence, du réservoir (36).

10. Dispositif selon la revendication 9, caractérisé par le fait que sur le bord libre de l'écran déflecteur (44) est formée une arête (40), que sur le bord intérieur de la garniture (42) est façonnée une autre arête (41) et que sur la surface interne de la paroi du diffuseur (45) est aménagée une arête d'égouttage (49) qui coopère avec les arêtes (40, 41) de manière qu'aux points de dérivation ainsi formés pour le jet d'aérosol, les plus grosses gouttes soient séparées de celui-ci et ramenées au produit de pulvérisation (16) contenu dans le réservoir (36).

# FIG. 1

## FIG. 2

# FIG. 3